# EUROPEAN PATENT APPLICATION

(11) **EP 4 702 975 A1**
(43) Date of publication of application: **04.03.2026**
(21) Application number: 25199692.2
(22) Date of filing: 02.09.2025
(51) Int. Cl.: A61K 9/16, A61K 9/50, A61K 31/7004, A61P 13/02, A23L 29/256, A23L 29/262, A23L 33/115, A23L 33/125, A23L 33/21

(54) **NUTRACEUTICAL COMPOSITION FOR THE TREATMENT OF CYSTITIS**

(30) Priority: 03.09.2024 IT 202400019579
(71) Applicant: BIOTECNICA DI MAGAGNINI MATTIA E MALATINI SILVIA SNC STP, 60022 Castelfidardo AN (IT); Manfredini, Milo, 41042 Fiorano Modenese MO (IT)
(72) Inventor: MANFREDINI, Milo, 41042 FIORANO MODENESE MO (IT); MAGAGNINI, Mattia, 60022 CASTELFIDARDO AN (IT)
(74) Representative: Modiano, Micaela Nadia

(57) **Abstract**

The present invention relates to a nutraceutical composition comprising D-mannose, one or more colloids based on hydroxypropyl methylcellulose (HPMC) and optionally hydroxyethyl cellulose (HEC), one or more of a vegetable oil and a wax, optionally an alginate, particularly for use in maintaining the correct function of the urinary tract and as an adjuvant in treating cystitis.

## Description

The present invention relates to a nutraceutical composition for maintaining the physiological function of the urinary tract and as an adjuvant in the treatment and prevention of cystitis.

The term "cystitis" refers to an inflammation of the bladder. Such inflammations can be of bacterial origin (bacterial cystitis), the most common, and non-bacterial (for example radiotherapy-induced cystitis and interstitial cystitis.

20%-30% of adult women develop one or more episodes of cystitis every year. Incidence increases with age: it is very low before puberty and increases with the beginning of sexual activity and pregnancy, and it continues to rise after menopause. Furthermore, relapses are more frequent as age increases.

The probability of recurrent cystitis rises with the increase in the number of previous episodes, while it drops as the interval between previous episodes lengthens.

The cause of recurrent cystitis lies in the bacterial reservoir constituted by fecal flora (gram-negative aerobic microorganisms). These bacteria, which are normally present in feces, under particular conditions can infect the lower urinary tract and so lead to cystitis. Of these, the biggest cause of cystitis is Escherichia coli (E. coli) (80% of cases) followed by Staphylococcus epidermidis (9%) and Streptococcus faecalis (1-3%).

Normally in a healthy subject there is, in particular in the urethra (lower urinary tract), an innocuous bacterial microflora that does not cause infections.

The most frequent infection mechanism in recurrent cystitis in women is "ascendant", meaning pathogenic bacteria travel from the periurethral zone to the urethra and then on to the bladder. This is a process by stages, wherein germs originating from the intestine colonize the vagina and the urethral mucous membrane and then give rise to a bladder infection.

Therefore, the principal cause of recurrent urinary infections in women is represented by the alteration of normal vaginal bacterial flora. It would also appear that the greater susceptibility of some women to recurrent cystitis must be sought in factors of genetic predisposition, which increase the possibility of bacteria adhering to the bladder wall. This would explain why only some women develop this disease even though almost all women are exposed to one or more of its causes.

This "genetic predisposition" negates one or more defense mechanisms that are normally present in the human urinary apparatus. Some of these are: the presence of glycosaminoglycans, which impede the adhesion of pathogenic bacteria to the bladder wall; the presence in normal quantities of non-pathogenic bacteria such as, for example, lactobacilli and gram-positive bacteria in the vagina and in the periurethral zone which impede the growth of pathogenic strains; the acidity (acid pH) of the vaginal environment and of urine prevent the proliferation of the bacteria responsible for cystitis; the presence in urine of a renal protein (Tamm-Horsfall protein), the function of which is to prevent the adhesion of bacteria to the bladder wall and to trap any bacteria that may be present so that they can be eliminated along with the urine; the presence of specific immunoglobulins (antibodies) on the bladder wall also present an effective barrier to colonization by bacteria.

The alteration or suppression of one or more of these factors increases the risk of recurrent infections.

Other risk factors are age, as mentioned previously; mechanical trauma resulting from sexual relations, which favors the reascent of pathogenic flora to the urethra; use of a diaphragm and spermicidal creams, which can alter the vaginal pH and therefore the pH of the normal bacterial flora of the vagina; a prolapse of the uterus or bladder, one of the consequences of which can be incomplete emptying of the bladder, which can favor the onset of cystitis; the same thing can be said for cases of neurogenic bladder dysfunction resulting from conditions such as, for example, multiple sclerosis, diabetes and spinal paralysis; an increased susceptibility to recurrent bladder infections can also be observed in all conditions of immunosuppression such as, for example, prolonged cortisone therapy and chemotherapy; finally, it must be remembered that calculi in the urinary tract is frequently associated with urinary infection.

There are three typical symptoms of cystitis:
- pollakiuria (increased frequency of urination);
- painful urination;
- urgency (an urgent and painful need to urinate).

Sometimes these symptoms may be accompanied by the presence of blood in the urine (haematuria), fever and chills.

Among the general measures to adopt to combat cystitis, it is important to get sufficient rest and to hydrate (drink more than 2 liters of water every 24 hours) so as to dilute the bacterial load present in the bladder.

Treatment must take into account that the pathogenic germs that are the most common cause of cystitis are in the vagina and have their natural reservoir in feces in the colon/rectum. Medicines must therefore also have an activity at the vaginal and intestinal level. Among the medicines currently on the market there are, for example, fluorquinolones, such as for example ciprofloxacin, trimethoprim/sulfamethoxazole and nitrofurantoin. However, recent studies show that fluorquinolones favor the onset of antibiotic-resistant strains, which result in ineffective treatment for relapses, and serious and irreversible side effects.

Nutraceuticals are used as an aid to treatment with antibiotics or to prevent relapse. Nutraceuticals are composed of botanical extracts, such as for example redcurrant extract, probiotics and specific sugars like D-mannose.

The latter represents one of the best aids to antibiotic treatment since D-mannose, once ingested, is poorly absorbed by the cells in the human body, and is therefore safe for use even by diabetics; the D-mannose is for the most part is eliminated, unmetabolized, in the feces and urine.

The action mechanism underpinning its anti-infective activity is represented by competitive inhibition of bacterial adhesion, in particular of E. coli (the main culprit of infections of the urinary tract). D-mannose in fact attaches to the adhesins of bacteria, proteins that enable the bacteria to "latch on" to the bladder wall, so preventing their radication. The high affinity of D-mannose for the adhesins of E. coli has also been shown for many other bacteria that have fimbriae, such as Pseudomonas aeruginosa. In this way, D-mannose facilitates the detachment of bacteria and their consequent elimination along with the normal urinary flow.

One of the principal drawbacks to treatment with nutraceuticals is linked to pharmacodynamics which, inevitably, mean that mannose-based nutritional supplements are eliminated along with urine, which therefore means there is a point in time when they are not present in the bladder and they interact with bacteria. This means it is necessary to take them continuously, for example in the acute phase or in cases of recurrent cystitis, every 2 or 3 hours at a dose of from 0.5-1 g per day, a factor that contributes to poor compliance with treatment by the patients and to the onset of side effects such as flatulence and diarrhea.

Conversely, a low frequency of taking mannose, for example 1 or 2 times per day, is rather ineffective in treating cystitis because the mannose is rapidly excreted and the adherence of the bacteria to the bladder walls is not effectively inhibited.

As part of this task, an object of the invention is to provide a nutraceutical composition that is easy to take in order to improve patient compliance with the treatment.

Another object of the invention is to provide a nutraceutical composition that makes it possible to reach and maintain a progressive high concentration of D-mannose in the urine and which provides an activity that inhibits the formation of biofilm for more extended periods than the compositions currently available, so reducing relapses of cystitis.

Another object of the invention is to provide a nutraceutical composition that is effective in preventing and treating cystitis, which is easily implemented using techniques that are commonly used in the sector.

### SUMMARY OF THE INVENTION

In light of the drawbacks described above, the aim of the present invention is to provide a nutraceutical composition that is effective in the inhibition of bacterial biofilm, in particular when induced by E. coli.

The aim and the objects of the invention are achieved by a slow-release nutraceutical composition of D-mannose that comprises D-mannose, at least one colloid or colloidal system based on hydroxypropyl methylcellulose (HPMC) and/or hydroxyethylcellulose and one or more lipids selected from a plant oil and/or a wax, wherein said composition is in the form of granules characterized by a particle size comprised between 40 µm and 2000 µm, preferably between 100 µm and 1500 µm, more preferably between 300 µm and 1400 µm measured by means of analytical sieving (Pharmacopoeia 2.9.38).

Preferably the colloid is based on hydroxypropyl methylcellulose (HPMC).

It has been observed that taking granulated mannose, or a composition that contains mannose as described herein, is effective in inhibiting and/or reducing the adherence of bacteria to the bladder walls for a longer time with respect to the products currently on the market, so reducing relapses and recovery times and increasing cases of remission from bacterial infection without resorting to the use of antibiotics.

The aims and objects of the invention are also achieved by the composition as described herein for use in maintaining the physiological function of the urinary tract and/or as an adjuvant in the prevention and/or treatment of cystitis and/or of a urinary infection by Escherichia coli bacteria.

According to another aspect, granules based on D-mannose are provided which comprise an outer coating for the delayed release of said D-mannose, wherein said granules comprise:
- HPMC and/or hydroxyethylcellulose,
- optionally one or more of hydroxyethyl cellulose and an alginate,
- lipids comprising waxes and a vegetable oil and/or fat,
wherein said granules have a particle size comprised between 40 µm and 2000 µm, preferably between 100 µm and 1500 µm, more preferably from 300 µm to 1400 µm, measured by analytical sieving.

Preferably the granules comprise a surface covering based on HPMC and/or hydroxyethylcellulose (HEC) and lipids comprising waxes and a vegetable oil, which covers the D-mannose at least partially.

According to certain aspects, the lipid components comprise waxes and plant oil which, as described herein, form a matrix that substantially embeds, at least partially, the D-mannose. Advantageously, the embedded D-mannose is released slowly from the lipid matrix, so providing a prolonged effect.

### DESCRIPTION OF THE FIGURES

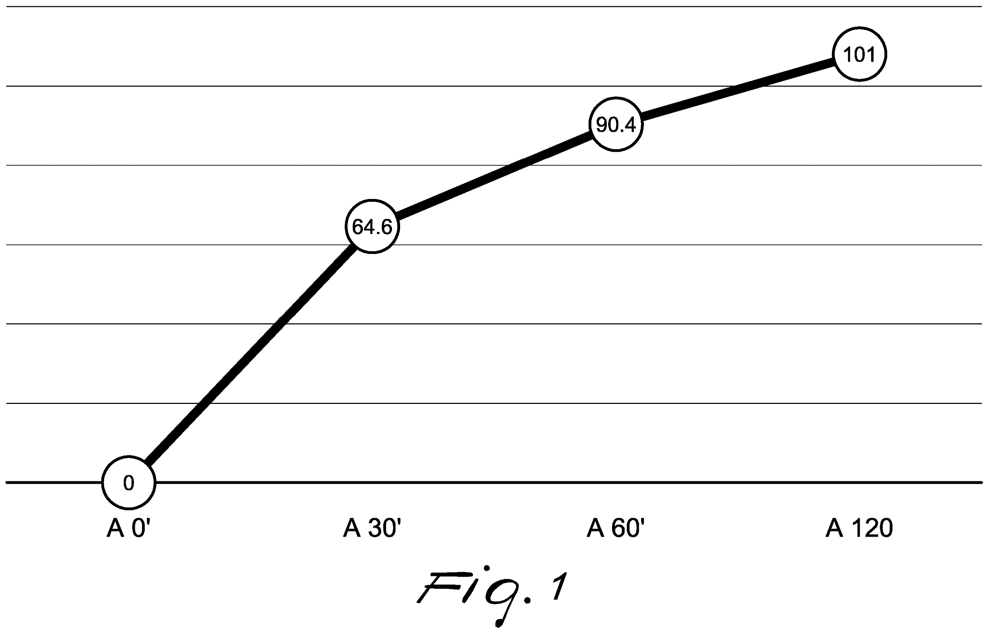

Figure 1 shows the curve of the release profile over time of D-mannose, expressed as a percentage, from capsules with an outer coating of HPMC according to Example 5 (sample A). The curve shows a rapid release of D-mannose, almost all of which is released within 2 hours;

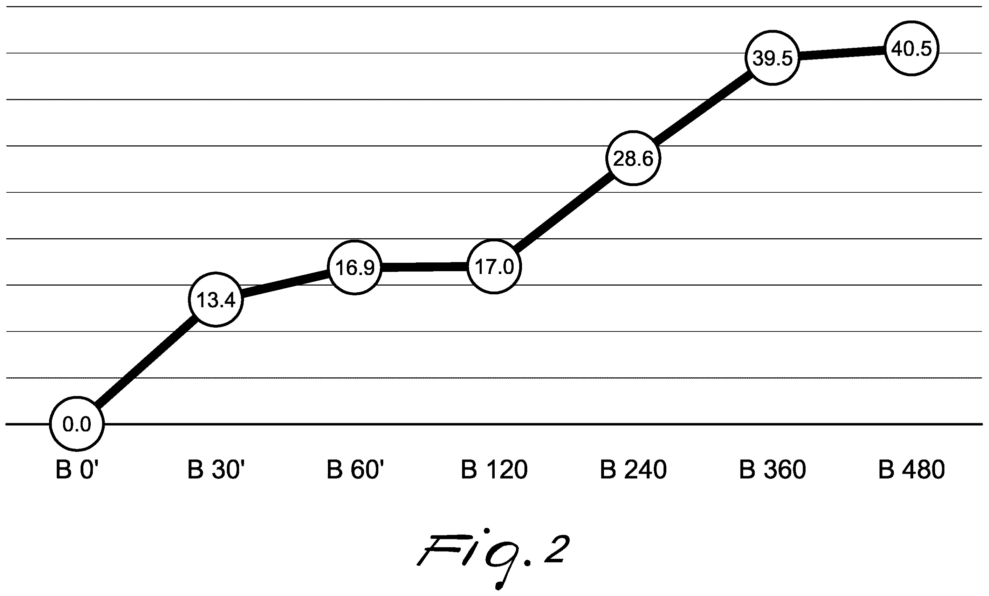

Figure 2 shows the curve of the slow release profile of D-mannose, expressed as a percentage, of an embodiment of the composition described herein, according to Example 5 (sample B). The curve shows a prolonged release of D-mannose from the formulation;

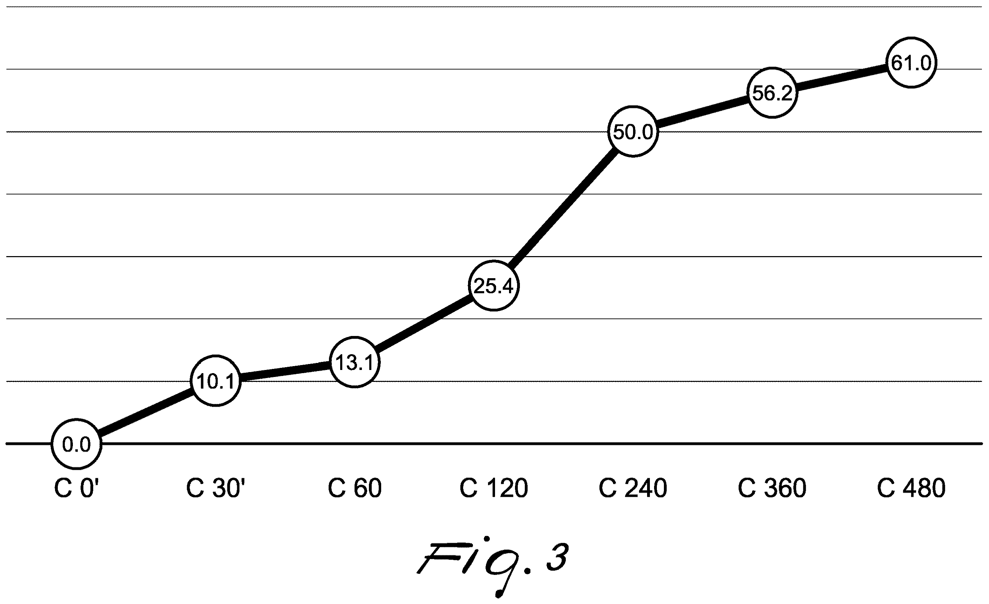

Figure 3 shows the curve of the release profile of D-mannose, expressed as a percentage, of an embodiment of the composition described herein, according to Example 5 (sample C). The curve shows a prolonged release of D-mannose from the formulation.

### DETAILED DESCRIPTION OF THE INVENTION

In a first aspect, the present invention relates to a prolonged release nutraceutical composition of D-mannose that comprises D-mannose, at least one colloid based on hydroxypropyl methylcellulose (HPMC) and/or hydroxyethylcellulose and one or more of a plant oil and a wax, wherein said composition is in the form of granules characterized by a particle size comprised between 40 µm and 2000 µm, preferably between 100 µm and 1500 µm, more preferably between 300 µm and 1400 µm measured by means of analytical sieving (2.9.38 Particle-size distribution estimation by analytical sieving, European Pharmacopoeia 11.0 met. 01/2010:20938).

Preferably said granules contain D-mannose in granules at least partially coated with a film or coating based on hydroxypropyl methylcellulose (HPMC) and/or hydroxyethylcellulose, and one or more lipids based on a vegetable oil and a wax.

Suitable oils include vegetable oils such as currant oil, olive oil, coconut oil, grape seed oil, medium-chain triglyceride (MCT) oil, rice oil, soya oil, oil of various seeds, sunflower oil, hydrogenated vegetable oils, stearin and/or animal oils such as fish oil or krill oil.

Preferred oils are of vegetable origin, preferably selected from currant oil, vegetable medium-chain triglycerides, coconut oil, stearin and mixtures thereof.

A suitable wax comprises a lipid mixture comprising saturated fatty acid esters of high molecular weight, for example from 10 to 40 carbon atoms, preferably from 10 to 30 carbon atoms and fatty alcohols, such as for example isopropyl or cetyl or glycol, for example such as ethylene glycol or propylene glycol, with two or more fatty acids of high molecular weight, for example from 10 to 40 carbon atoms.

Suitable fatty alcohols comprise alcohols with a chain of from 3 to 26 carbon atoms, for example from 12 to 24 carbon atoms or from 16 to 18 carbon atoms.

A suitable wax comprises a mixture of esters of fatty acids and fatty alcohols.

Typically the wax is a physiologically acceptable wax, adapted for oral administration, for example a vegetable wax or an animal wax.

Examples of suitable waxes include glyceryl monostearate (GMS), glyceryl dibehenate;
vegetable waxes, for example carnauba wax, castor wax, myrtle wax, jojoba wax, ouricury wax, rice wax, Japanese wax, esparto wax;
animal waxes, for example beeswax, spermaceti, lanolin; palmitate-based waxes, for example: myricyl palmitate, palmitic acid, hydroxypalmitates and oleate esters with long chains, for example from 30 to 32 carbon atoms, long chain aliphatic alcohols and fatty acids, saturated or unsaturated, waxes derived from petroleum for example paraffin waxes, alpha olefins.

The composition in granules containing D-mannose covered as described herein achieves an effect of slow, prolonged release of the D-mannose, and of longer-lasting inhibition of the bacterial biofilm induced by E. coli.

According to certain aspects, the composition described herein is a delayed release composition of D-mannose for the prolonged release of D-mannose after oral administration.

Preferably the composition can further contain hydroxyethylcellulose and/or alginates as substances that further prolong the release times of the D-mannose from the coated granules.

Preferably, the composition of the invention comprises D-mannose in an amount comprised between 50% and 90%, preferably between 75% and 85% by weight on the total weight of the composition.

Preferably, the composition of the invention comprises HPMC and/or hydroxyethylcellulose, in an amount comprised between 5 and 50%, preferably between 10% and 20%, on the total weight of the composition.

Preferably, the composition of the invention comprises one or more of a wax and/or a vegetable oil in an amount comprised between 0.01 and 15%, preferably 0.1% and 10%, on the total weight of the composition.

In a preferred embodiment the composition of the invention comprises D-mannose in an amount comprised between 75% and 85%, HPMC in an amount comprised between 10% and 20%, and one or more of a wax and a vegetable oil in an amount comprised between 0.1% and 10%, each on the total weight of the composition.

Advantageously, the ethyl cellulose or hydroxyethylcellulose and the alginate can be present in the composition, for example in an amount comprised between 1% and 10%, preferably between 2% and 4% by weight on the weight of the composition, in order to have a greater release time of the mannose and obtain a prolongation over time of the effect of the mannose.

The composition in granules according to the invention can be obtained through "fluid bed" granulation, which uses a fluidization process, a unit operation that converts fine particles to a pseudo-fluid state using a flow of gas. At certain limits of speed, the fluid is capable of keeping the particles suspended, and the particles are free to move within the flow. A fluidized bed is comparable to a boiling liquid: the solid particles are subject to heavy turbulence, which increases with the speed of the gas. Fluid bed granulation is a process that forms the granules in a single apparatus, wherein the binder is atomized on a bed of fluidized powders in one or more passes (alternating steps of atomization and drying).

Comparisons made between fluid bed granulation and traditional techniques have found finer and more homogeneous granules.

According to some embodiments, the air in the process is first treated in a system comprising a pre-filter, a heat exchanger, preferably pre-heated, a dehumidifier, a rehumidifier, and a high-efficiency antiparticulate filter. In many applications external air is used which, for pharmaceutical operations, must be free from dust and pollutants.

The air, once converted to optimal conditions for the process, is distributed in the powder bed. This operation is delicate: an uneven distribution of air can result in channeling of the flow, and to an inadequate fluidization. Preferably, for a suitable fluidization, both the powder container and the air distributor are optimized for the specific preparation.

According to some embodiments, the method of preparation of the granules entails that, before being inserted in the fluid bed, the D-mannose is loaded into a mixer and the lipid phase is added under stirring to the liquid and/or solid phase.

Subsequently the HPMC-based binder solution is atomized in the mixing chamber. Advantageously, during the step of drying in the fluid bed, the fatty phase surfaces, forming a layer that coats the D-mannose with the formation of granules. Preferably the step of drying is performed on a fluid bed.

In this way the principal effect is achieved, of modifying the release profile of the D-mannose granule which, in the composition of the invention, is at least partially covered by a coating based on hydroxypropyl methylcellulose, waxes and oils. Preferably, the nutraceutical composition according to the invention further comprises one or more ingredients chosen from an additional alginate, polyethylene glycol (PEG), and glycerol.

According to some aspects, in the process of production of the granules, in a first step the HPMC component of the binder solution interacts with D-mannose in powder form until the binder solution has substantially solvated the granules, subsequently the wax is added which, at the end of the drying process, floats to the surface to form a film that at least partially coats the granule based on HPMC and D-mannose.

In a preferred embodiment the composition described herein can comprise currant oil.

In a second aspect, the present invention relates to the composition according to the invention for use in maintaining the physiological function of the urinary tract and as an adjuvant in the prevention or treatment of cystitis or of a urinary infection by Escherichia coli bacteria.

According to another aspect of the invention, granules containing D-mannose are supplied in granule form comprising an outer coating for the delayed release of said D-mannose, wherein said outer coating comprises:
- HPMC,
- optionally one or more of hydroxyethyl cellulose and an alginate,
- waxes and a vegetable oil,
wherein said granules have a particle size comprised between 300 µm and 1400 µm measured by means of analytical sieving (Pharmacopoeia 2.9.38).

Advantageously, said granules can include any characteristic according to any of the embodiments described herein.

According to some preferred embodiments, said granules contain a core of mannose which is at least partially covered with a coating containing hydroxypropyl methylcellulose, a wax and a vegetable oil according to any embodiment described above. Preferably, the granules of D-mannose form a core covered by the coating components based on hydroxypropyl methylcellulose, waxes and oils and optionally an alginate and ethyl cellulose.

According to some embodiments, the composition containing D-mannose or the granules of D-mannose described herein are, or form an agglomerate with the oils and waxes according to any embodiment described herein. According to these aspects, an agglomerate is supplied for the protracted release of D-mannose which comprises D-mannose, hydroxypropyl methylcellulose and/or hydroxyethylcellulose, a lipid selected from oil and waxes, and mixtures thereof according to any embodiment described herein.

The invention will now be described with reference to the following non-limiting examples.

### EXAMPLE 1

Formulation of a composition according to the invention.

Granule with particle size of 1000 µm for the delayed release of D-mannose, comprising
Granule of D-mannose 80%
coated by a coating that comprises
hydroxypropyl methylcellulose 15% by weight
waxes and oil 5% by weight.

### EXAMPLE 2

In-vitro test to determine the effect of inhibiting the formation of biofilm ("test solution 1").

To assess the effect of inhibiting the formation of biofilm, 1L of a first test solution "test solution 1" is prepared:
- 6.8 g of Eugon,
   formula of eugon:
   pancreatic digest of casein 15.0 g/l
   pancreatic digest of soybean meal 5.0g/l
   L-Cystine 0.7 g/l
   Sodium chloride 4.0 g/l
   Sodium sulfite 0.2 g/l
   Glucose 5.5 g/l
   pH 7.0 ± 0.2 at 25°C
- 1.2 g of Peptone,
- 6.8 g of sodium chloride,
- 10 mL of an 0.5 McFarland infective solution obtained from revitalization of the bacterial strain of E. Coli, and
- water, sufficient amount 1000 mL.

The Escherichia coli titer of test solution 1 thus composed is 10⁶-10⁷ UFC E. coli/ml.

The test solution 1 was divided into 4 aliquots of 200 ml and placed in sterile bottles made of Pyrex glass.

Starting from the four aliquots, the four following compositions were prepared:
a1) 200 mL of test solution 1 + 4 g of pure D-mannose in powder form (D-mannose 98%);
b1) 200 mL of test solution 1 + 4 g of D-mannose granulate obtained using a 10% solution of HPMC, followed by calibration with sieving in order to obtain a particle size between 300-1400 µm and drying in a static oven. Final formula D-mannose 90% - HPMC 10%;
c1) 200 mL of test solution 1 + 4 g of composition according to the invention (D-mannose was granulated by spraying the 20% solution of HPMC, followed by drying and calibration with a sieve in order to obtain a particle size comprised between 300-1400 µm, then the mixture of waxes/vegetable oil was sprayed and subsequently cooled. The oil used is currant oil but other vegetable oils can also be used for this purpose, in that the oil itself is used to regulate the melting point and retard solidification at the moment of addition of the wax to the granule, and is quantitatively dependent on the composition of the waxes themselves).

The final formula is the following: D-mannose 75-85% - HPMC 10-20% - waxes/vegetable oil 0.1-10%.

d1) 200 mL of test solution 1.

The solutions were incubated at 37°C, and aliquots were taken at time zero and after 2.5 hours, after 4 hours and after 6 hours, on which the tests of inhibition of biofilm were conducted.

### EXAMPLE 3

In-vitro test to determine the effect of inhibiting the formation of biofilm ("test solution 2").

To demonstrate that the biofilm inhibition effect observed in the first test (see Table 1) was due neither to the enriched medium, nor to a concentration effect of formulations of D-mannose, a "test solution 2" was prepared which did not contain Eugon, and lower quantities of D-mannose were used in the various formulations. 1L of a second test solution "test solution 2" was prepared:
- 1.0 g of Peptone,
- 10 mL of an 0.5 McFarland infective solution obtained from revitalization of the bacterial strain of E. Coli, and
- water, sufficient amount 1000 mL.

The E. coli titer of the test solution thus composed is 10⁶-10⁷ UFC E. coli/ml.

The test solution 2 was divided into 4 aliquots of 200 ml in sterile bottles made of Pyrex glass.

Starting from the four aliquots, the four following compositions were prepared:
a2) 200 mL of test solution 2+ 1.5 g of commercial D-mannose in powder form supplied by Rial Pharma S.r.l.;
b2) 200 mL of test solution 2 + 1.5 g of D-mannose granulate obtained using a 10% solution of HPMC, followed by calibration with sieving in order to obtain a particle size between 300-1400 µm and drying in a static oven. Final formula D-mannose 90% - HPMC 10%;
c2) 200 mL of test solution 2 + 1.5 g of composition (D-mannose was granulated by spraying the 20% solution of HPMC, followed by drying and calibration with a sieve in order to obtain a particle size comprised between 300-1400 µm, then the mixture of waxes/vegetable oil was sprayed and subsequently cooled. The oil used is currant oil but other vegetable oils can also be used for this purpose, in that the oil itself is used to regulate the melting point and retard solidification at the moment of addition of the wax to the granule, and is quantitatively dependent on the composition of the waxes themselves).

The final formula is the following: D-mannose 75-85% - HPMC 10-20% - waxes/vegetable oil 0.1-10%.

d2) 200 mL of test solution 2.

The solutions were incubated at 37°C, and aliquots were taken at time zero and after 1 hour, after 3.5 hours, after 4 hours, after 5 hours and after 6 hours, on which the biofilm inhibition tests as described in Example 4 were conducted.

### Example 4

Assessment of biofilm in the solutions of Examples 2 and 3.

The aliquot of each solution taken at each time interval (2400 µL) was placed in a spectrophotometer cuvette (Shimatsu UV 1280). 600 µL of water was added to each test tube.

The cuvettes were then incubated in darkness at 37°C overnight.

After 24 hours, the supernatant was removed from each cuvette, and the cuvettes containing the biofilm were rinsed with sterile water.

After washing, staining for biofilm was performed by adding Crystal Violet for 10 minutes. The dye attaches to the biofilm present in the cuvette, giving it a characteristic violet color. After 10 minutes of staining the cuvette was rinsed again with running water and left to dry.

Once dried, a semi-quantitative assessment of the formation of biofilm was obtained by extracting the Crystal Violet with 3 mL of the following solution: 200 mL methanol, 50 ml glacial acetic acid and 250 mL water.

This solution dissolved the bonded Crystal Violet and produced a violet-stained solution.

The intensity of the staining was determined by measuring the absorbance at 570 nm.

The results for solution 1 (prepared in Example 2) are given below in Table 1:

| | T 0 | T 2.5 | T 4 | T 6 |
|---|---|---|---|---|
| a1 | 0.088 | 0.142 | 0.157 | 0.162 |
| b1 | 0.098 | 0.131 | 0.144 | 0.154 |
| c1 | 0.101 | 0.141 | 0.101 | 0.112 |
| d1 | 0.089 | 0.129 | 0.132 | 0.142 |

The results in Table 1 indicate that the solution obtained from the granular composition according to the invention (c1) up until 2.5 hours has a lesser biofilm inhibition effect than the other forms but, with the passage of time, the inhibition effect is greater than all the other forms. This result is compatible with its characteristic of having a delayed release with respect to pure D-mannose (used in a1).

Furthermore, when compared to the granulated form, the effect of the solution on the biofilm is greater. In particular, the analytic result shows that the solution obtained with the coated granule according to the invention, shows a lower absorbance with respect to the granulated one. This result is in line with the principle of the method, whereby absorbance values are inversely proportional to efficacy, i.e. the most efficacious value has the lowest absorbance.

In all the forms, c1 is the solution that at the end of the 6h interval showed a substantially lower level of biofilm.

The results for solution 2 (prepared in Example 3) are given below in Table 2:

**Table 2:**

| | T 0 | T 1 | T 3.5 | T 4 | T5 | T 6 |
|---|---|---|---|---|---|---|
| a2 | 0.044 | 0.068 | 0.078 | 0.082 | 0.085 | 0.092 |
| b2 | 0.048 | 0.047 | 0.058 | 0.063 | 0.072 | 0.087 |
| c2 | 0.052 | 0.081 | 0.075 | 0.062 | 0.068 | 0.071 |
| d2 | 0.048 | 0.052 | 0.072 | 0.070 | 0.072 | 0.078 |

The results in Table 2 show that the solution obtained from the granular composition according to the invention (c2), although before 3.5 hours is less efficacious than the other compositions in inhibiting adhesion of biofilm, from 4 hours onward displays a greater inhibition effect than all the other forms on the adhesion of biofilm.

The data shown in Table 1 and 2 show that the modified-release composition in the form of granules according to the invention, although in the initial hours of the test it does not have a superior efficacy to that of pure D-mannose (a1 and a2) nor of granulated D-mannose (b1 and b2), with the passage of time it displays a greater efficacy against the adherence of biofilm.

This observation aligns with a dose mediated effect of pure D-mannose, which in the initial hours of the test has a greater inhibition effect but which tends to decrease as the free mannose bonds with the bacterial adhesins during the bacterial replication phase.

Therefore, the mannose in the composition according to the invention, released slowly, displays a greater efficacy over time against biofilm precisely because of its characteristic release profile.

### Example 5

### Comparative test

Some comparative tests were conducted of time-release D-mannose by compositions according to embodiments of the invention, with respect to a conventional pharmaceutical form.

The release times of D-mannose from a traditional pharmaceutical form constituted by a capsule of HPMC containing D-mannose (Sample A) were compared with the release times of D-mannose formulated according to embodiments of the invention identified as "20250624-007 test 7" (Sample B) and "20250624-008 test 8" (Sample C).

### Materials and Methods

The release profile was simulated by using a dissolution test, following the parameters given below:
- number of revolutions equal to 70±2 revolutions per minute;
- temperature of 37 ± 2 °C.
- test times: 8 hours divided into two phases;
- HCl 0.1N pH 1.78: 2 hours
- Phosphate buffer pH 6.80: 6 hours.

Both the sample (A) constituted by a capsule of HPMC containing D-mannose in powder form and the two samples (B and C) based on D-mannose according to two embodiments were first of all weighed and then placed in 1000 ml containers for the dissolution test.

Aliquots of buffer were collected at regular intervals to verify the content of D-mannose.

After 2 hours, the HCl 0.1 N buffer was substituted with phosphate buffer at pH 6.8 and the test continued for a further 6 hours, for a total duration of 8 hours.

During the first two hours in the acid buffer, the following sampling was conducted: at 30, 60 and 120 minutes.

During the remaining 6 hours in the phosphate buffer, the following sampling was conducted: at 240, 360 and 420 minutes of contact with the phosphate buffer at pH 6.8.

At each time point, 2 ml was taken and each volume taken was immediately replaced with 2 ml of HCl or phosphate buffer, according to the phase of the test, in order to maintain a constant volume in each container.

The content of D-mannose was determined by following the method described in the publication "Effects of monosaccharide composition on quantitative analysis of total sugar content by phenol-sulfuric acid method".

The principle of determination of the method phenol-sulfuric acid method is that concentrated sulfuric acid dehydrates polysaccharides to form uronic acid and hydroxyurea formaldehyde, and then condenses with phenol to form red-orange compounds. We assessed the spectrum of absorption of a solution of D-mannose at 40 mg/l and observed a peak in absorption around 490nm, which represented the wavelength used for the spectrophotometric measurements.

Before the measurements, the samples collected over the period of 8 hours were suitably diluted in order to fall within the concentration range of our calibration curve (0, 20, 40, 50, 100 mg/L). The colorimetric development was executed by adding 1 mL of 6% phenol solution, and 5 mL of 98% sulfuric acid to 2 mL of each sample, as described in the publication cited above.

In parallel to the analysis of the release of D-mannose, we also assessed the amount of HPMC released over time by the same samples.

The method used to produce the HPMC is based on the USP 36 method.

The quantities of D-mannose measured in the various phases were reduced by the corresponding HPMC values, so obtaining a normalized result which was not potentially influenced by releases of HPMC.

Below is a table summarizing the results of the test.

Composition of the samples subjected to testing:
Sample A: background-art capsule containing D-mannose with HPMC coating;
Sample B: agglomerate according to an embodiment of the invention containing HPMC 12%; lipids (waxes and oils) 5%, D-mannose 79%.
Sample C: agglomerate according to an embodiment described herein containing HPMC 12%; lipids (waxes and oils) 10%, D-mannose 83%.

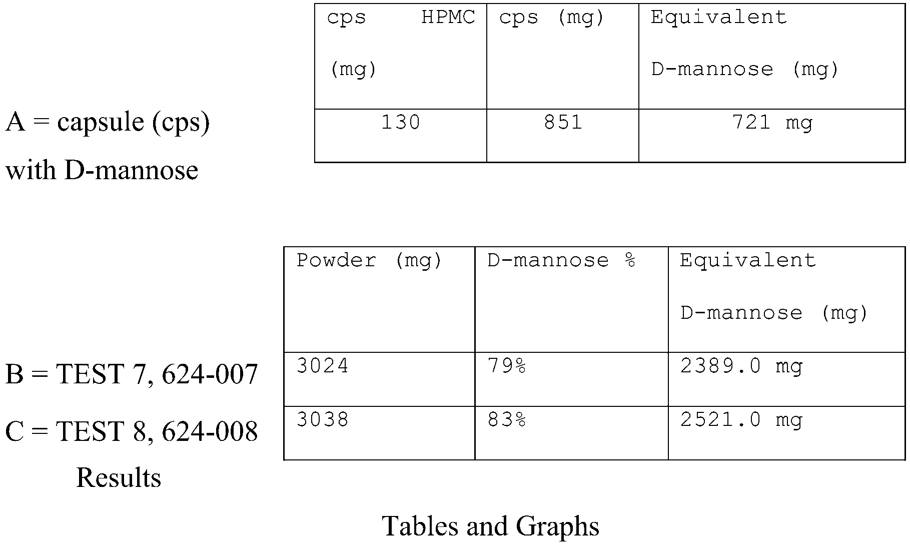

Sample A: D-mannose release capsules

| Time (t) min | Release D-mannose (mg) | Release D-mannose % |
|---|---|---|
| 0 | 0 | 0 |
| 30 | 466.15 | 64.6 |
| 60 | 652.05 | 90.4 |
| 120 | 779.25 | >100 |

It was observed that after 2 hours of exposure in HCl 0.1 N, the capsule containing D-mannose dissolved completely. Therefore, the subsequent step of dissolving in phosphate buffer was not conducted.

Sample B TEST 7 (20250624-007): release of D-mannose (mg) (HCl buffer 0.1 N for 120 minutes plus phosphate buffer pH 6.8 for a further 360 minutes. Total duration of the test: 480 minutes)

| | Time (t) min | D-mannose release (mg) | D-mannose release (%) |
|---|---|---|---|
| HCl 0.1 N | 0 | | |
| | 30 | 320.07 | 13.4 |
| | 60 | 403.2 | 16.9 |
| | 120 | 406.1 | 17.0 |
| Phosphate buffer pH 6.8 | 240 | 683.99 | 28.6 |
| | 360 | 942.4 | 39.5 |
| | 480 | 967.25 | 40.5 |

Sample C TEST 8 (20250624-008): Release of D-mannose (mg) (HCl buffer 0.1 N for 120 minutes plus phosphate buffer pH 6.8 for a further 360 minutes. Total duration of the test: 480 minutes)

| | Time (t) min | D-mannose release (mg) | D-mannose release (%) |
|---|---|---|---|
| HCl 0.1 N | 0 | 0 | 0.0 |
| | 30 | 255.73 | 10.1 |
| | 60 | 331.395 | 13.1 |
| | 120 | 639.55 | 25.4 |
| Phosphate buffer pH 6.8 | 240 | 1261.75 | 50 |
| | 360 | 1416.8 | 56.2 |
| | 480 | 1538.55 | 61.0 |

### Conclusions

The results of the comparative test show that the formulations of Samples B and C containing HPMC, waxes and D-mannose according to embodiments of the invention have a very different release profile from that of the formulations in capsules, based on HPMC.

The outer enclosure in HPMC of traditional capsules does not modulate the release of D-mannose and it releases substantially all of the D-mannose contained within two hours.

These experimental results are consistent with the guidelines of the European Pharmacopoeia XX Edition, which gives a dissolution time of 2 hours for solid pharmaceutical forms that are not slow-release.

By contrast, the composition according to embodiments described herein has a prolonged effect of D-mannose release longer than 4 hours.

This delayed release effect of D-mannose considerably reduces the adhesion of bacteria to the bladder walls and reduces the bacterial load of the bladder for a prolonged time, so allowing an efficacious treatment of the most common forms of cystitis, including recurrent forms.

The disclosures in Italian Patent Application No. 102024000019579 from which this application claims priority are incorporated herein by reference.

## Claims

1. A slow-release nutraceutical composition of D-mannose, which comprises D-mannose, at least one colloid based on hydroxypropyl methylcellulose and/or hydroxyethylcellulose, and a lipid based on vegetable oil and a wax, wherein said composition is in the form of granules **characterized by** a particle size comprised between 40 µm and 2000 µm, preferably between 100 µm and 1500 µm, more preferably between 300 µm and 1400 µm, measured by analytical sieving.

2. The nutraceutical composition according to claim 1, wherein said composition further comprises at least one alginate and preferably one or more of polyethylene glycol and/or glycerol.

3. The nutraceutical composition according to claim 1 or 2, wherein the D-mannose is in an amount comprised between 50% and 90%, preferably between 75% and 85% by weight on the total weight of the composition.

4. The nutraceutical composition according to any one of the preceding claims, comprising HPMC and/or hydroxyethylcellulose in an amount comprised between 5% and 50%, preferably between 10% and 20%, by weight on the total weight of the composition.

5. The nutraceutical composition according to any one of the preceding claims, wherein the one or more of a vegetable oil and a wax is in an amount comprised between 0.01% and 15%, preferably between 0.1% and 10%, by weight on the total weight of the composition.

6. The nutraceutical composition according to any one of the preceding claims, wherein said composition comprises one or more of hydroxyethylcellulose and an alginate in an amount comprised between 1% and 10%, preferably between 2% and 4%, by weight on the total weight of the composition.

7. The nutraceutical composition according to any one of the preceding claims, wherein said composition comprises currant oil.

8. Nutraceutical composition according to any one of the preceding claims for use in maintaining the physiological function of the urinary tract and/or as an adjuvant in the prevention and/or treatment of cystitis and/or of a urinary infection by Escherichia coli bacteria.

9. The composition for the use according to claim 8, wherein said cystitis is recurrent cystitis and said urinary infection by Escherichia coli bacteria is a recurrent infection.

10. Granules for the prolonged release of D-mannose, which contain D-mannose in granule form comprising an outer coating for the delayed release of said D-mannose, wherein said outer coating comprises:
- HPMC and/or hydroxyethylcellulose,
- optionally at least one alginate,
- waxes and a vegetable oil,
wherein said granules have a particle size comprised between 40 µm and 2000 µm, preferably between 100 µm and 1500 µm, more preferably from 300 µm to 1400 µm, measured by analytical sieving.

11. The granules according to claim 10, wherein said outer coating partially coats the D-mannose in granule form.
